# EUROPEAN PATENT SPECIFICATION

(11) **EP 2 393 928 B1**
(45) Date of publication and mention of the grant of the patent: **20.11.2013**
(21) Application number: 10703455.5
(22) Date of filing: 08.02.2010
(51) Int. Cl.: C12N 15/82

(54) **USE OF TREHALASE TO OBTAIN DROUGHT RESISTANCE IN PLANTS**
VERWENDUNG VON TREHALASE ZUR ERZIELUNG VON WIDERSTANDSFÄHIGKEIT GEGEN TROCKENHEIT BEI PFLANZEN
UTILISATION DE TRÉHALASE EN VUE D'OBTENIR UNE RÉSISTANCE À LA SÉCHERESSE DANS DES VÉGÉTAUX

(30) Priority: 09.02.2009 US 207188 P
(43) Date of publication of application: 14.12.2011
(73) Proprietor: VIB VZW, 9052 Gent (BE); Katholieke Universiteit Leuven, K.U. Leuven R&D, 3000 Leuven (BE)
(72) Inventor: VAN DIJCK, Patrick, B-3271 Zichem (BE); VANDESTEENE, Lies, B-3010 Kessel-Lo (BE); AVONCE, Nelson, B-3000 Leuven (BE)
(86) International application number: PCT/EP2010/051500
(87) International publication number: WO 2010/089392

(56) References cited:
- EP-A1- 1 111 051
- WO-A1-96/21030
- US-A1- 2005 160 501
- GAMEZ-ESCOBEDO ANALI ET AL: "Trehalase Gene Silencing in Plants: Hidric Stress Tolerance and Biomass Increase" PLANT BIOLOGY (ROCKVILLE), vol. 2008, 2008, page 137, XP009131353 & JOINT ANNUAL MEETING OF THE AMERICAN-SOCIETY-OF-PLANT-BIOLOGISTS/SOCI EDAD-MEXICANA-DE-BIOQUIMICA; MERIDA, MEXICO; JUNE 26 -JULY 01, 2008
- KAWANO N (REPRINT) ET AL: "Trehalase-inhibited transgenic tobacco plants by using antisense genes of trehalase increase abiotic stress tolerance." 2004, PLANT AND CELL PHYSIOLOGY, VOL. 45, SUPP. [S], PP. S228-S228. ISSN: 0032-0781. PB - OXFORD UNIV PRESS, GREAT CLARENDON ST, OXFORD OX2 6DP, ENGLAND. , XP009131337 abstract
- JANG IN-CHEOL ET AL: "Expression of a bifunctional fusion of the Escherichia coli genes for trehalose-6-phosphate synthase and trehalose-6-phosphate phosphatase in transgenic rice plants increases trehalose accumulation and abiotic stress tolerance without stunting growth." PLANT PHYSIOLOGY (ROCKVILLE), vol. 131, no. 2, February 2003 (2003-02), pages 516-524, XP002574546 ISSN: 0032-0889 cited in the application
- LOPEZ MIGUEL ET AL: "Trehalose and trehalase in root nodules of Medicago truncatula and Phaseolus vulgaris in response to salt stress" PHYSIOLOGIA PLANTARUM, vol. 134, no. 4, December 2008 (2008-12), pages 575-582, XP002574547 ISSN: 0031-9317

## Description

The present invention relates to the use of trehalase to obtain drought and/or salt resistance in plants. More specifically, it relates to genetically modified constructs, transformed into plants and resulting in overexpression of trehalase, whereby the transformed plants show a significantly better drought resistance during the drought period, and a better recovery when water is supplied after the drought period.

The disaccharide trehalose (α-D-glucopyranosyl-1,1-α-D-glucopyranoside) is a non-reducing sugar, found in a variety of organisms, including plants. The role of trehalose has often been associated with stress resistance, particularly resistance to desiccation. Jang et al. (2003) describe the construction of transgenic rice plants with increased abiotic stress tolerance, by the expression of a fusion of the *Escherichia coli* genes for trehalose-6-phosphate synthase and trehalose-6phosphate phosphatase. Suarez et al. (2008) obtained improvement of drought tolerance in common beans by overexpression of trehalose-6-phosphate synthase. In those studies, the accumulation of trehalose is promoted by increasing the biosynthesis.

However, apart from the biosynthetic pathway, the level of trehalose may be influenced by trehalose degradation. Trehalase is the only known enzyme in Arabidopsis, able to degrade trehalose to two glucose units. The expression of its gene, *AtTRE1,* is significantly upregulated during specific plant development stages such as flowering and senescence and during stress conditions. Because of the intimate connection between trehalose metabolism and plant vital processes, stringent level control of trehalose, or indirectly of T6P, by trehalase activity might be crucial in the fine regulation of these metabolites/signals and subsequently, in the control of plant growth and development. Trehalase may also mobilize stress-induced trehalose levels to allow subsequent cellular repair, as is the case in lower organisms. Moreover, high trehalase activity may also prevent perturbations of carbon metabolism by external trehalose, released by pathogenic or symbiotic microbes. However, despite the involvement in multiple processes, little is known about the exact role of trehalase in higher plants.

EP0784095 discloses trehalose accumulation in potato upon inhibition of trehalase, either by addition of the trehalase inhibitor Validamycin A, or by expressing trehalase anti-sense RNA. However, these inhibition experiments were carried out in trehalose-6-phosphate synthase overexpressing plants, making it difficult to make a distinction between the effect of the overexpression of the synthase and that of the inhibition of the trehalase. WO2008095919 discloses the use of overexpression of a trehalase gene to confer nematode resistance to plants. However, no reference to drought or salt tolerance was made. On the contrary, it was indicated that higher trehalose concentration results in increased tolerance to drought and stress.

Surprisingly we found that overexpression of trehalase resulted in a better tolerance of drought, and a better growth upon rewatering after a period of drought. Knock out of the trehalase gene caused a decrease in drought tolerance.

A first aspect of the invention is the use of recombinant trehalase overexpression to obtain drought and/or salt tolerance in plants.

Trehalase as used here means a glycoside hydrolase, converting trehalose into glucose. Trehalases are known to the person skilled in the art, and include, but are not limited to bacterial, fungal and plant trehalases. Preferably, said trehalase is an *Arabidopsis trehalase,* even more preferably it is the *Arabidopsis trehalase TRE1* (genbank accession number NM_118536). A recombinant trehalase, as used here, means that the nucleic acid encoding the trehalase has been isolated, was optionally modified, and introduced into a plant, resulting in modified drought and/or stress tolerance. Said nucleic acid may have an optimized coding sequence. Optimized coding sequences, as used here, includes, as a non limiting example, codon optimation for better translation as well as mutations that may increase the specific activity of the protein. However, modification of the nucleic acid is optional. As a non-limiting example of the use of a trehalase without modification, the gene of one species, even a non-plant species, may be introduced in the plant. Said introduction can be a single copy, or several copies can be introduced. Alternatively, the plant may be transformed with the endogenous gene to obtain a higher copy number of the gene, and so a higher expression of the trehalase protein. Drought tolerance, as used here, comprises drought tolerance during cultivation, as well as drought tolerance after harvesting, resulting in a better shelf life of the harvested plant. As a non limiting example, drought tolerance after harvest may result in a better shelf life of ornamental flowers, or in a better shelf life of vegetable, preferably green vegetables such as, but not limited to, lettuce and spinach.

Preferably, said overexpression of said trehalase is specifically in the stomatal guard cells. Specific expression in the stomatal guard cells as used here means that the expression is largely biased towards the stomatal guard cells; preferably there is expression in the stomatal guard cells and no expression in the mesophyllic or epidermal cells of the plant leafs or plant stem, but there may be expression in parts of the root or the flowers. Such specific overexpression can be realized by the endogenous *Arabidopsis thaliana TRE1* promoter.

Methods to obtain recombinant trehalase overexpression are known to the person skilled in the art, and include, but are not limited to increasing the copy number of trehalase genes, placing the trehalase coding sequence under control of a strong promoter, or removing repressing elements from the promoter. Preferably, trehalase overexpression is obtained by isolating a trehalase gene, adapting it for overexpression and transforming it into a plant. Preferably, said overexpression is resulting in improved drought tolerance. Preferably said trehalase is placed after a stomata specific promoter, and said overexpression is specifically in the stomatal guard cells. Still another aspect of the invention is the use of trehalase overexpression to obtain improved growth upon rewatering after a drought period. Rewatering, as used here, means that after a period of drought, the plant is exposed again to water. Improved growth as used here includes, amongst others, a shorter adaptation period after the start of the rewatering, and better growth after adaptation.

Still another aspect of the invention is the use of a recombinant trehalase overexpression according to the invention, to limit the aperture of the stomatal cells, and/ or the number of stomatal cells per leaf surface unit, and/or the stomatal index. Preferably, modulation of the stomatal function is realized by modulating the aperture of the stomata. Preferably said use of trehalase is overexpression and said modulation of the stomatal function is a limitation of the opening of the stomata. It is clear for the person skilled in the art that a limitation of the opening of stomata, or and/or a decrease in number of stomatal guard cells per leaf surface, or and/or a decrease of stomatal index can limit water loss and result in drought resistance, at least as long as a decrease of one factor is not compensated by an increase of another factor.

### BRIEF DESCRIPTION OF THE FIGURES

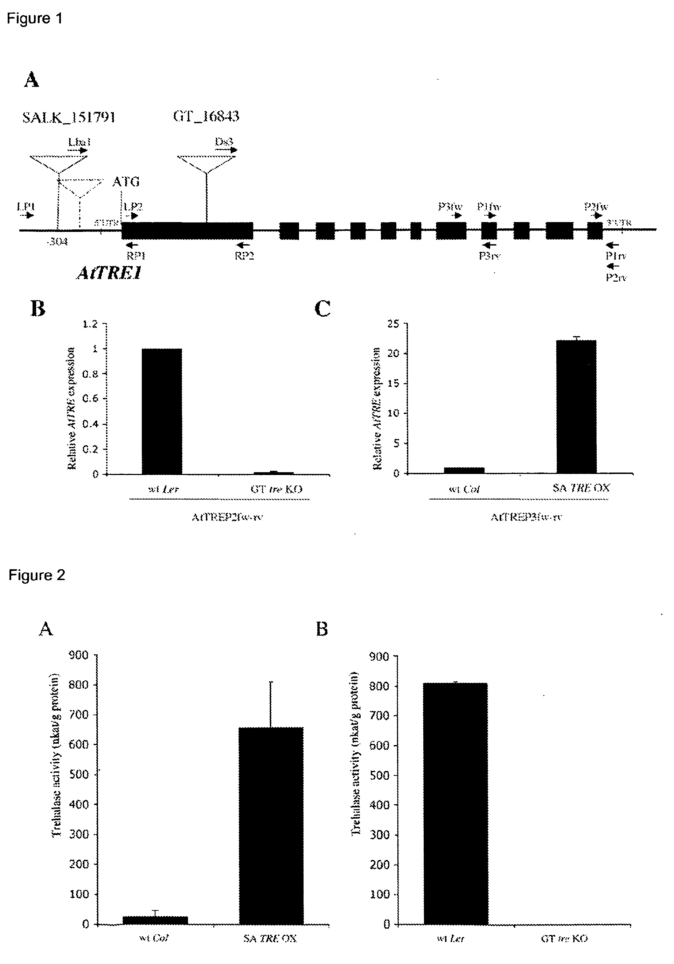

**Figure 1****: Arabidopsis *AtTRE1* mutant lines. A)** Cartoon of the insertion sites of GT *tre* KO and SA *TRE* OX lines in gDNA of Arabidopsis. The exact (full lines) and predicted (dotted lines) insertion sites of SA *TRE* OX are shown. For GT *tre* KO, prediction insertion site was confirmed. Positions of primer sets are also shown; black boxes are exons. **B)** Relative trehalase expression of GT *tre* KO lines, compared to wild type (wt) *Arabidopsis thaliana* ecotype *Landsberg erecta (Ler)* **C)** Relative trehalase expression of SA *TRE* OX lines, compared to wt *Arabidopsis thaliana* ecotype *Colombia (Col).* For B) and C) real-time PCR results normalized to actin, are shown from one representative line. Homozygous seedlings were grown for 10 days on 1 xMS (1% suc, 12h-12h day/night cycle). Data are means ± SE, n=2, with ± 100 seedlings per experiment.
**Figure 2****: Trehalase activity of *AtTRE1* Arabidopsis mutant lines.** Trehalase activities (nkat/g protein) were determined **A)** in adult leaves of wt *Col* and SA *TRE* OX plants, grown in soil; **B)** in mixtures of young and ripe silique material of wt Ler and GT *tre* KO, grown in soil (data are mean values of mixed plant material from different plants per line).
**Figure 3****: Growth phenotypes of *AtTRE1* mutants on trehalose. A)** GT *tre* KO lines and wt *Ler* with Val A. **B)** SA *TRE* OX lines and wt *Col* with Val A. **C)** GT *tre* KO lines and wt Ler without Val A. **D)** SA *TRE* OX lines and wt *Col* without Val A. Approximately 30 seedlings/treatment were grown on vertical plates, 1xMS without suc, in a 12h-12h day/night cycle, for 10 days with 0, 0.5, 1, 5, 10, or 25 mM trehalose. Data are means ± SE.
**Figure 4****: Growth phenotypes of *AtTRE1* mutants on high trehalose.** Seedlings grown in same conditions as described in Fig. 3, without Val A, and with higher concentration of trehalose: 50, 100, 150, and 200 mM. Representative seedlings are shown.
**Figure 5****: Carbohydrate use of *AtTRE1* mutant seedlings. A)** SA *TRE* OX plants and wt Col plants grown on horizontally oriented plates with 1xMS, 0%, 3%, 6% suc or glc, 16h-8h, 10 days. **B)** GT *tre* KO plants and wt Ler plants (same conditions as in A). **C)** SA *TRE* OX plants and wt *Col* plants grown on vertically oriented plates in continuous light with 1% sucrose, 0.5xMS, 6 days. **D)** GT *tre* KO plants and wt Ler plants (same conditions as in C). Representative seedlings are shown.

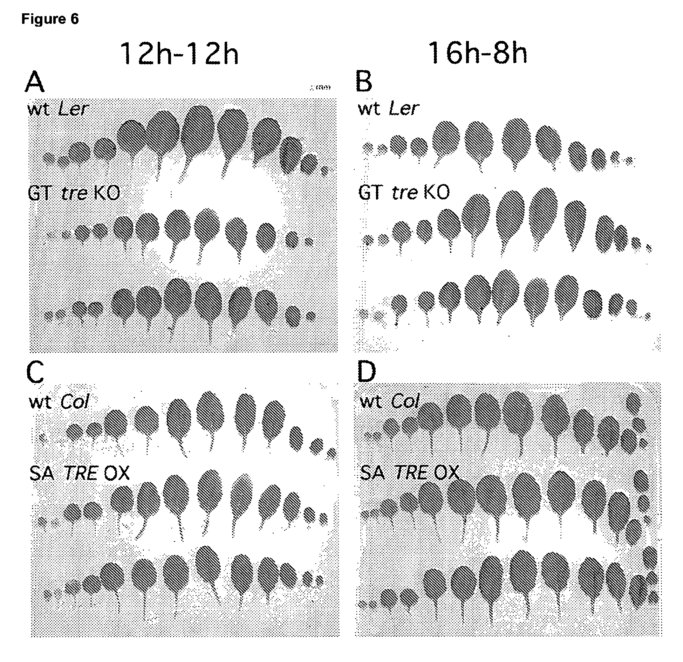

**Figure 6****: Leaf growth of *AtTRE1* mutants at different diurnal rhythm. A)** Leaves of wt Ler (first line) and GT *tre* KO plants (two different lines), 12h-12h day/night cycle. **B)** Leaves of wt *Ler* (first line) and GT *tre* KO plants (two different lines), 16h-8h day/night cycle. **C)** Leaves of wt *Col* (first line) and SA *TRE* OX plants (two different lines), 12h-12h day/night cycle. **D)** Leaves of wt *Col* (first line) and SA *TRE* OX plants (two different lines), 16-8h day/night cycle. Cotelydons, and rosette leaves are placed in order of appearance. Plants 4 weeks old, grown in soil. Representative seedlings are shown.
**Figure 7****: Reproduction of *AtTRE1* mutants. A)** Earlier flowering of GT *tre* KO plants compared to wt Ler plants, grown in soil, 4 weeks old (16h-8h). **B)** View of plant development of wt *Ler* and GT *tre* KO plants and **C)** wt *Col* and SA *TRE* OX plants, grown in soil for 7 weeks old (16h-8h). **D)** Detail of flowers and siliques of wt *Ler* and GT *tre* KO plants. Phenotypes were observed several times, and representative plants are shown.
**Figure 8****: Seedling development of *AtTRE1* mutants at elevated temperature.** Germination and growth of SA *TRE* OX and GT *tre* KO seedlings and respectively wild types in 33°C (continuous low light, 1xMS, 1% suc, 10 days). Representative seedlings are shown.
**Figure 9****: Drought responses of *AtTRE1* OX and KO Arabidopsis plants.** Drought stress responses of plants, grown in soil, in normal watered conditions for 4 weeks, stopped watering for 2 weeks (A, B) and a couple of days after rewatering (C, D). Representative plants are shown.
**Figure 10****: Localization of AtTRE1 in mesophyll protoplasts. A)** Light microscopy image of protoplast using dark interference contrast. **B)** Localisation of AtTRE1 with GFP-fluorescence mainly in the plasma-membrane. **C)** The auto-fluorescence of chloroplasts.
**Figure 11****: *AtTRE1* expression pattern in 10 D old seedlings (grown on 1x MS), using promoter-GUS/GFP single insertion lines after GUS staining.**
   A: expression in leaf A, leaf 2 and leaf primordia; B: expression in stomatal guard cells.
**Figure 12****: Stomata closure and opening assay of AtTRE1 OX and KO lines.** Differences in opening of stomata in trehalase overexpression lines (OX) compared with the trehalase knock out (KO) and the wild type plants.
**Figure 13****: Stomata and pavement cell counting assay of *AtTRE1* OX and KO lines.** Number of stomata (A) and pavement cells (B) counted on a sample of 0.142mm² for trehalase overexpressing plants (OX) compared with the knock out line (KO) and the wild type plants
**Figure 14****: Stomatal index of *AtTRE1* OX and KO lines.** The stomatal index corresponds to the ratio of stomata per total number of cells in the epidermis (#stomata/(2x #stomata+#pavement cells).

### EXAMPLES

### Materials and methods to the examples

### Arabidopsis plants used in this study

**Table I: plant lines**

| **Name** | **Ecotype** | **Source** |
|---|---|---|
| SA *TRE* OX (SALK_151791) | *Colombia* | ABRC* |
| GT *tre* KO (GT_16843) | *Landsberg erecta* | CSHL* |

| | | |
|---|---|---|
| * From the ordered lines, homozygous plants were obtained in this study | | |

### Primers used in this study

**Table II: Cloning and side-directed mutagenesis primers**

| **Primer** | **Sequence** | **RE** |
|---|---|---|
| AtTRE1fw | GGAAGATCTATGAAATCATACAAACTTAATAACC | *Bgl*II |
| AtTRE1rv | TCCCCCGGGGGCTTCAATGCTAAGATGAGAG | *Sma*I |
| AtTRE1717sdmfw | AACTATCTTGTCTACAAGAAAAGTGGGACTA | |
| AtTRE1717sdmrv | TAGTCCCACTTTTCTTGTAGACAAGATAGTT | |

| | | |
|---|---|---|
| abbreviations: fw (forward), rv (reverse); RE (restriction enzyme). | | |

**Table III: Real-time PCR primers and semi quantitative RT-PCR primers**

| **Name primer** | **Sequence** |
|---|---|
| AtTREP1fw | ACATGGAAGGCTGAGAACC |
| AtTREP1rv | CCAAGAAATAATCCACTACG |
| AtTREP2fw | CCTCTCATCTTAGCATTGAAG |
| AtTREP2rv | CCAAGAAATAATCCACTACGAT |
| AtTREP3fw | AGCGAGAGAGAAAGCGTTTC |
| AtTREP3rv | CCTTCCATGTCTCAGATTCC |
| ACTIN2fw | GAAGAGCACCCTGTTCTTCT |
| ACTIN2rv | TGGCGTACAAGGAGAGAACA |

**Table IV: Primers to check insertion in Arabidopsis mutants**

| **Primer** | **Sequence** | **Mutant line** |
|---|---|---|
| Lba1 | TGGTTCACGTAGTGGGCCATCG | SALK |
| Ds3 | CCGTCCCGCAAGTTAAATATG | GT |
| AtTRELP1 | GCCTTTTTCAAACTCAAGTTGCAC | SA *TRE* OX |
| AtTRERP1 | GAGTTTATTATGTGTGTGCGTGGA | SA *TRE* OX |
| AtTRELP2 | ACTCATCTCCACGCACACAC | GT *tre* KO |
| AtTRERP2 | AATCTCGAACCGGGAATGAT | GT *tre* KO |

| | | |
|---|---|---|
| Abbreviations: SA (SALK lines); GT (Genetrap line), LP (left primer), and RP (right primer). | | |

### Plasmids used in this study

Trehalase CDS was cloned (source material: silques and flowers of wild type *Arabidopsis thaliana* ecotype *Colombia*) in plant vector pGFP (Kovtun *et al.,* 1998; 2000; Hwang and Sheen, 2001). pGFP= pUC18 modified for GFP fusion. CDS sequence was blasted against TAIR Arabidopsis Genome Initiative Coding Sequence datasets (www.arabidopsis.org) and mutations, leading to amino acid changes, were corrected by side-directed mutagenesis (sdm).

### Growth media and growth conditions

### Vapor-phase sterilization of Arabidopsis seeds

Transgenic and wild type Arabidopsis, ecotype *Col* and ecotype *Ler seeds* were vapor-phase sterilized according to a protocol adapted from Ye *et al.* (1999). Seeds were placed in open micro-centrifuge tubes and put inside a desiccator jar, where bleach-chlorine fumes sterilize the seeds for a period of 3-9h (bleach + concentrated HCl). Sterile water is added afterwards working in laminar flow, followed by a 48h cold treatment under constant light for imbibition and stratification.

### Growth in soil

Vapor-sterilized seeds were sown, after a 48h stratification period, on wet soil and germinated for two days in high humidity. In general, they were grown in 12h-12h, 16h-8h day/night cycle or continuous light (21°C, 60% humidity, 100µmol s⁻¹ m⁻²).

### In vitro growth

Vapor-sterilized seeds were sown after a 48h stratification period in vertically or horizontally oriented Petri dishes on a full strength (or mentioned otherwise) Murashige and Skoog Salt Mixture including vitamins (MS) medium (4.3g/l) with MES (0.5g/l) and solidified with Phytagar (8g/l) (Duchefa, Haarlem, The Netherlands). The media was enriched with sucrose/glucose (0-6%) or trehalose, depending on the tested conditions, which are specified. Val A was added in a concentration of 10µM.

### Selection of plant transformants and homozygous plant lines

For homozygous plant selection, SALK lines, and GT lines (with the *NTPII* marker gene as selection marker) were screened on 1xMS + Kanamycin (50µg/ml, Sigma-Aldrich). For growth on MS media with selective media, seeds were germinated for 7 days on MS and antibiotics, positives were transferred to soil to flower and to set seeds.

### Molecular, physiological and biochemical techniques

### RNA extraction from plants and cDNA preparation

Plant samples (seedlings) were harvested and immediately frozen in liquid nitrogen. RNA extraction of plant material was performed with Trizol reagent (Invitrogen), according to the manufacturer's instructions. For each sample, reverse transcription (RT) was performed using 1µg of total RNA (Reverse Transcription System, Promega).

### Plasmid DNA isolation from bacterial cells

Mini preparation - CTAB method (Del Sal et al., 1988)
Mini preparation - GenElute™ Plasmid Miniprep kit (Sigma-Aldrich)
Maxi preparation - Pure Yield Plasmid Maxiprep System (Promega)
Rapid Genomic DNA isolation from Arabidopsis plants

After grinding a medium size leaf with screwdriver-pestle in 100µl TPS buffer (100mM Tris-HCl, pH 9.5, 1 M KCI, 10mM EDTA), the samples were incubated at 70°C for 20-30 min. The debris was pelleted by centrifugation at top speed for 10 min at RT and supernatans transferred to a new eppendorf tube. For further PCR reactions, 0.5 to 1µl supernatans was used in a 20-50µl reaction.

### Polymerase Chain Reactions (PCR)

### Standard PCR reactions

PCR reactions were performed using different polymerase kits, according to manufacturer's recommendations; High-Fidelity Taq Polymerases (Roche), Phusion High-Fidelity DNA polymerase (FINNZYMES) and Ultra Pfu HF DNA polymerase (Stratagene) for cloning and ExTaq (TAKARA) for diagnostic PCR.

### Purification of PCR products

Equal amount of PCI was added to the PCR product (45µl-45µl), vortex, and centrifuged at max speed, for 5 min. To the transferred supernatans, NaAc 3M (3.6µl) and ice-cold pure ethanol (109µl) was added, vortex, and spinned down for 1 min at max speed. After additional wash with ice-cold 80% ethanol, the pellet was dried out and dissolved in 12.5µl H₂O. PCR products could be also purified with using the Wizard SV Gel and PCR Clean-UP System (Promega, USA), according to manufacturer's guideline.

### Semi-quantitative RT-PCR

PCR was performed on cDNA samples equally diluted to 250ng/µl and a control gene was used as control. The PCR product was run on 1% agarose gel and visualised with SYBR Safe DNA gel stain (Invitrogen)

### Real-time PCR

25µl real-time PCR reaction was composed of 12.5µl Platinum SYBR Green qPCR SuperMix-UDG (Invitrogen), 0.5µl of ROX reference dye, 1.25µl of each primer of 500nM stock solution and 5µl of cDNA mix (100ng/µl). The PCR program comprised an initial denaturation of 2 min at 95°C, amplification by 50 cycles of 15s at 95°C, 1 min at 58°C The expression levels were analyzed with ABI prism and were all normalized to *ACTIN2* expression.

### Side-directed mutagenesis (sdm)

Side-directed mutagenesis (sdm) was performed according to the Stratagene's QuickChange Site-Directed Mutagenesis kit. As such, point mutations, insertions and deletions were created in a plasmid with Ultra Pfu HF DNA polymerase (Stratagene).

### Transient expression in Arabidopsis mesophyll protoplasts

Three to four week-old Arabidopsis plants (in soil, 12h-12h day/night cycle, under low light, 50-75µmol m⁻² s⁻¹) with healthy, unstressed, and well-expanded true leaves were used. This technique is detailed described by Yoo *et al.,* (2007) (http://genetics.mgh.harvard.edu/sheenweb/protocols reg.html). Briefly, after the removal of cell walls, protoplasts are released, washed and collected. DNA-PEG4000(platelets, Fluka)-Calcium transfection is performed using 2x10⁴ protoplasts per 10-20µg of endotoxin free (maxiprep) plasmid DNA. The transfected protoplasts are incubated for 6h in the light before harvesting.

Subcellular localization in mesophyll protoplast cells of the protein of interest can be determined by fluorescence microscopic analysis, using the GFP-tagged vectors.

### Trehalase measurements in plant material

Crude leaf material (300mg, mix of plant material/different plants) was homogenized and powdered on dry ice, suspended in 2ml ice-cold extraction buffer (0.1 M MES/K⁺, pH 6; 1 mM PMSF 2mM EDTA, 10mg/gFW insoluble PVP, polyvinylpyrrolidone). The suspension was vortexed and centrifuged (13000 rpm, cold, 10 min). The supernatans was dialysed (BRL microdialysis system) ON at 4°C against MES buffer pH 7 with 50µl CaCl₂. Trehalase activity was determined by a method described by Pemambuco *et al.* (1996) adapted to microtiter plates. 10µl dialysed sample was added to 50µl trehalose buffer (250mM trehalose, 25mM MES, pH 7, 50µl CaCl₂). The microtiter plate was incubated for 30 min at 30°C and reaction was stopped by boiling 5 min at 95°C warm waterbath. Glucose was determined using glucose oxidase-peroxidase method by adding 200µl GOD-PAP (Dialab) (blancs: residual glucose content in each sample determined by boiling samples before trehalose addition). A505 was measured after 15 min of incubation at 30°C. The amount of protein was determined using Lowry method with BSA as standard (Lowry *et al.,* 1951).

### Example 1: Isolation of Arabidopsis AtTRE1 mutants

The transposon insertion Genetrap (GT) line 'GT_16843' (*A. thaliana,* ecotype Ler), was originally produced in the Martienssen's Cold Spring Harbor Laboratory (Sundaresan *et al.,* 1995; Martienssen, 1998). Based on the flanking sequences of the insertion site obtained by TAIL-PCR (Liu *et al.,* 1995), line 'GT_16843' was predicted to carry a unique insertion of a GT transposable Ds element, 395 bp downstream of the ATG start codon, in the middle of the first exon of the *AtTRE1* gene (Fig. 1A). T3 descendants showed 3:1 (resistant-susceptible) segregation on KAN selection media, consistent with a single insertion. Homozygous lines were obtained and confirmed by PCR (Ds3, AtTRELP2-RP2 primer set) and the CSHL predicted insertion site was confirmed. Semi-quantitative RT-PCR (primerset AtTREP1fw-rv) and real-time PCR (primerset AtTREP2fw-rv, Fig. 1 B) revealed strong downregulation of *AtTRE1* in the homozygous 'GT_16843' plants, hereinafter designed as 'GT *tre* KO' plants.

The 'SALK_151791' line (A. *thaliana,* ecotype *Col*) (Fig. 1A), originated from the SALK Institute (Alonso *et al.,* 2003), was available from the Arabidopsis Biological Resource Center (ABRC), and ordered via NASC. From the ordered seeds, we selected plants, homozygous for a single T-DNA insertion in the *AtTRE1* gDNA. After KAN selection, plants were checked for homozygousy with primers Lba1 and AtTRELP1-RP1 primer set (designed with SIGnAL). Flanking T- DNA insertion sequences were obtained 304 bp upstream of the start codon (TAIR predicted 211 bp in front of the ATG start codon). Surprisingly, semi-quantitative RT-PCR (AtTREP1fw-rv) revealed higher *AtTRE1* expression in the mutants, suggesting OX instead of KO. This was confirmed by real time-PCR (Fig. 1C, homozygous SALK_151791 plants are hereinafter designed as SA *TRE* OX). Closer inspection of the left border of the T-DNA reveals a 35S CaMV promoter, which might be responsible for this overexpression. Also insertion of the T-DNA in an important region for transcription regulation (e.g. a repressor binding site) is possible.

Measurements of trehalase activity in different plant tissues demonstrated that SA *TRE* OX plants exhibited much higher trehalase activity than control wild type plants. For example, up to 25 times more trehalase activity was observed in adult leaves of *TRE* OX, compared to wild type *Col* leaves (Fig. 2A). In contrast, very low to no detectable trehalase activity was measured in the leaves and siliques (Fig. 2B) of the GT *tre* KO plants compared to the control plants.

### Example2: Phenotypic characterization of AtTRE1 mutants

Trehalose causes dramatic effects on plant metabolism, growth and development. Validamycin A (Val A) specifically inhibits trehalase activity, and prohibits the use of trehalose as an extra source of glucose. It is worth noting that, while Val A treatment alters adult plant stress and reproduction responses and even causes phytotoxicity, no phenotypic irregularities are observed in seedlings (Wingler *et al.,* 2000; Müller *et al.,* 2001; Ishikawa *et al.,* 2005; 2007; Ramon *et al.,* 2007).

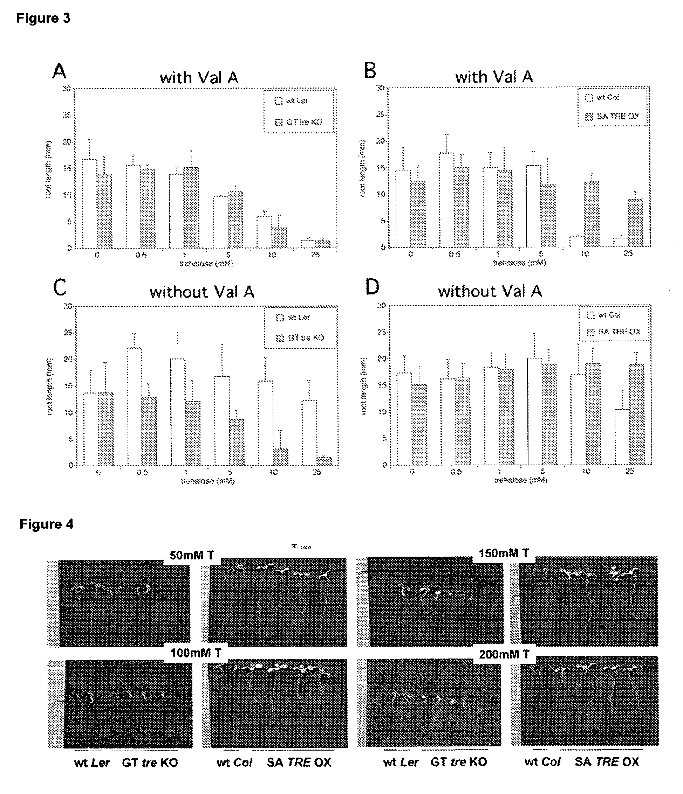

In our growth assay, no extra sugar was added to the media. As known from literature, gradually increased levels of trehalose in the presence of Val A, cause increased inhibition of root growth and development of wild type seedlings (Fig. 3A, B). The sensitivity to trehalose differed between the ecotypes Ler and *Col,* with a rather gradual inhibitory effect in Ler wild type plants, and a more dramatic decrease in root length of *Col* wild type plants at 10 mM trehalose. GT *tre* KO lines exhibited similar growth inhibition on trehalose as wild type Ler seedlings in the presence of Val A (Fig. 3A), consistent with lack of trehalase activity in both plants. In contrast, SA *TRE* OX plants were significantly more resistant to higher trehalose levels than wild type controls in the presence of Val A (Fig. 3B), suggesting that the amount of Val A (10 µM) is not sufficient to inhibit increased trehalase activity in the OX lines.

In the absence of Val A, external supplied trehalose can be used as an extra glucose source for growth. On high trehalose concentrations, wild type seedlings arrest with development shortly after germination, show altered cotyledons extension, and develop only short primary roots (Wingler *et al.,* 2000).

Interestingly, at low trehalose contents, GT *tre* KO seedlings do not seem to be able to use the extra glucose source for growth (Fig. 3C), because of total lack of trehalase activity. Moreover, with increasing trehalose concentrations, KO mutants are significantly more affected in development, compared to wild type Ler controls (Fig. 3C, Fig. 4). In contrast, SA *TRE* OX plants seem to be insensitive to high trehalose concentrations compared to wild type seedlings (Fig. 3D, Fig. 4).

Mutants in trehalose metabolism have been reported to exhibit sensitivity to supplemented sugars, and T6P has been highlighted as an important regulator for carbohydrate utilization (Schluepmann *et al.,* 2003; Avonce *et al.;* 2004).

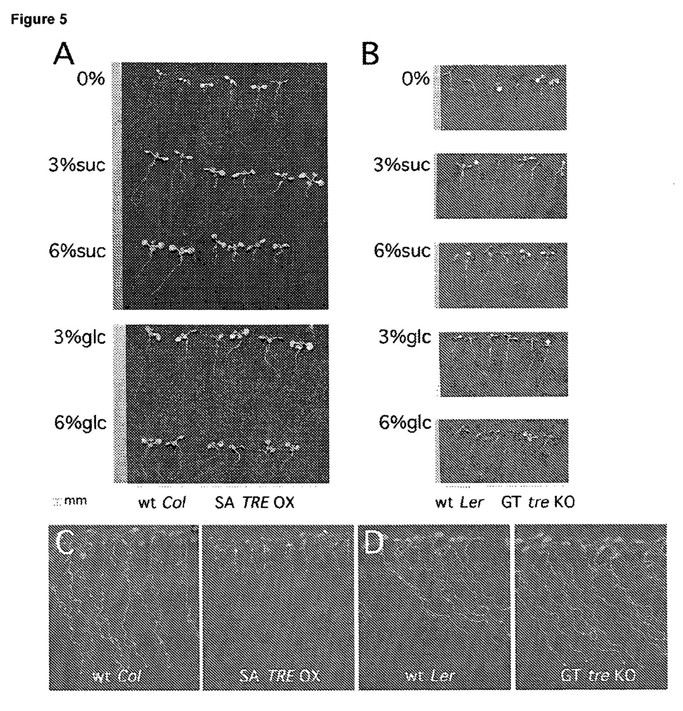

When grown on gradually increasing sugar concentrations, SA *TRE* OX seedlings exhibit similar leaf and hypocotyl development as control seedlings (horizontally orientated plates, 16h-8h day/night cycle (Fig. 5A). However, root growth of SA *TRE* OX seedlings was significantly affected, with reduced length and increased root branching. This aberrant phenotype was also observed on vertically oriented plates in continuous light with 1% sucrose (Fig. 5C). However, the mutants displayed a somewhat accelerated root growth on media without sugars. All together, it seems that overexpressing trehalase allows seedlings to efficiently use carbon supplies in low energy conditions, but makes them unable to deal with high energy conditions. In contrast, a slightly longer root growth of GT *tre* KO could be observed when more energy was available (Fig. 5B, D), and interestingly, GT *tre* KO plants seemed to germinate slower in media without sucrose.

Plants engineered in trehalose metabolism typically exhibit dramatic phenotypic irregularities, with altered physiology and morphology. Also Val A treatment can alter plant reproduction and can even cause phytotoxicity (Müller *et al.,* 1995; Müller *et al.,* 2001; Ishikawa *et al.,* 2005; 2007).

However to our surprise, altering endogenous trehalase activities did not seem to cause severe phenotypic irregularities in Arabidopsis. We did notice a slower germination rate of GT *tre* KO seeds in soil in a 12h-12h day/night cycle, and this phenotype was abrogated when increasing the light period to 16h. Moreover, in a 12h-12h day/night cycle, leaves of the GT *tre* KO plants were smaller (Fig. 6A), and when increasing the day length, the growth rate of these KO plants increased (Fig. 6B, growth stages according to Boyes *et al.* (2001), 1.10 for wt and 1.11 for KO).

This observation again suggests that growth of plants, altered in trehalase activity, strongly depends on the available energy. GT *tre* KO plants seem to have problems to cope with low energy conditions, whereas higher growth rates are achieved when more energy is available, e.g. through longer photosynthesis or supplied sugars. In contrast, SA *TRE* OX plants grow slightly better than controls in low carbohydrate conditions (Fig. VI.6C, e.g. rosette leaf nr 9), while at a 16h-8h day/night cycle, no obvious growth phenotype was observed (Fig. 6D).

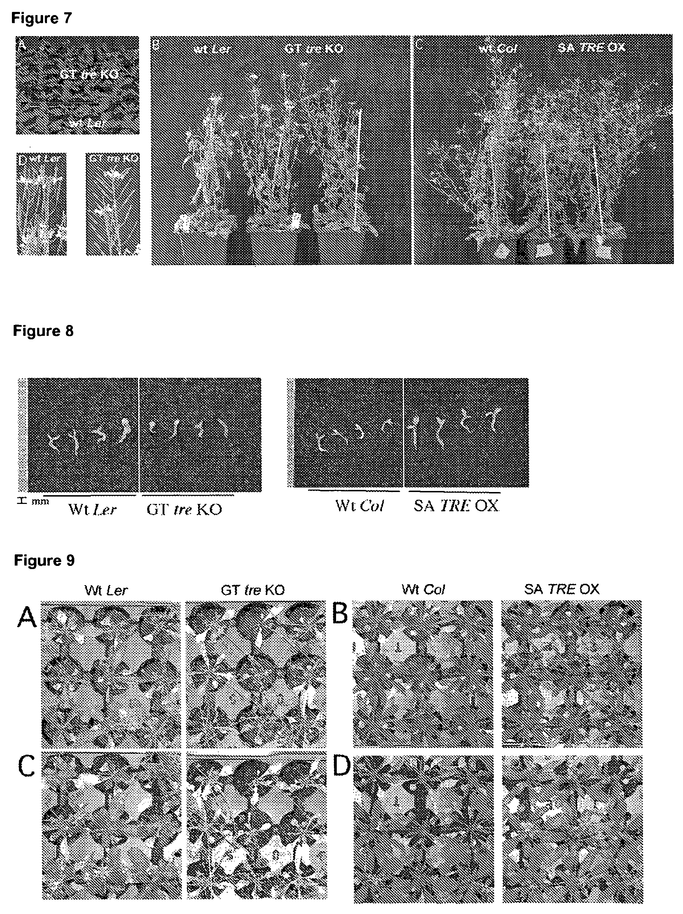

Interestingly, GT *tre* KO plants started flowering earlier than wild types (16h-8h day/night cycle) (Fig. 7A), displayed longer inflorescence stems, and altered silique orientation (Fig. 7B;D). Stems of SA *TRE* OX plants (Fig. 7C) appeared somewhat smaller, but no obvious other phenotypes could be observed.

### Example 3: Trehalase and stress responses

Given trehalose modified plants performed better in multiple stress treatments, we tested trehalase OX and KO plants under variable stress conditions.

When performing heat-stress during germinating (33°C, continuous low light, 1xMS, 1% suc), we noticed a better early seedling development of SA *TRE* OX seeds, compared to wild type seeds, in contrast to GT *tre* KO lines (Fig. 8).

These results, together with ample circumstantial evidence of pivotal roles of trehalose metabolism in abiotic stress responses, give us incentives to look closer to more environmental stresses such as drought and dehydration stresses in the vegetative stage. Four-weeks old KO and OX plants grown in a 16h-8h day/night cycle (in soil with controlled watering regime), were subjected to drought stress for 2 weeks and then rewatered. In contrast to the performances of GT *tre* KO lines, the SA *TRE* OX plants withstand and recovered much better the dehydration stress (Fig. 9).

### Example 4: AtTRE1 gene expression analysis and subcellular localization

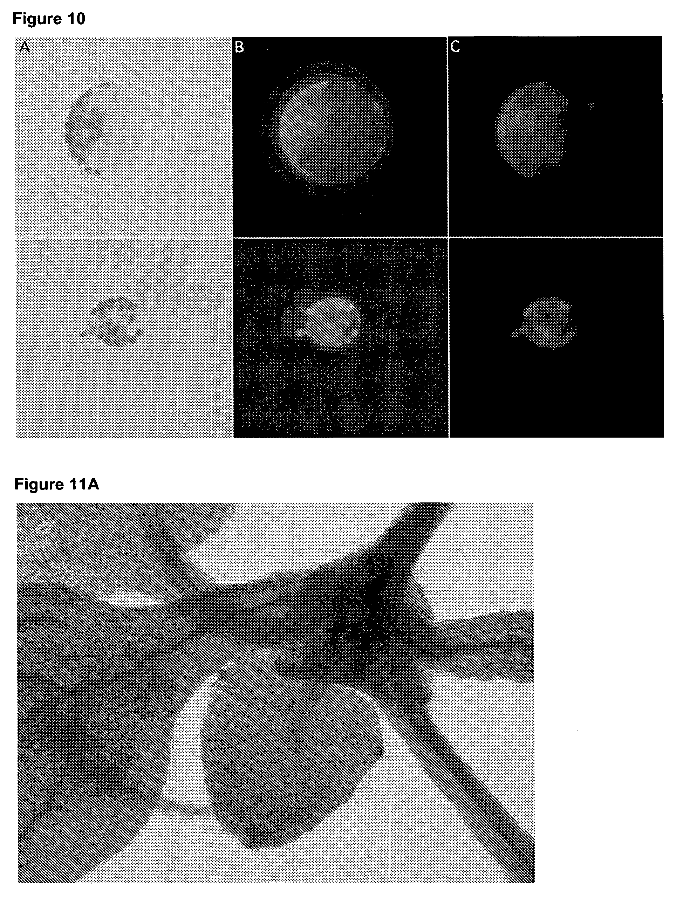

Given trehalase controls extracellular trehalose levels, but seems also to regulate plant trehalose (sugar) metabolism, we analysed the fluorescence of AtTRE1-GFP constructs in Arabidopsis mesophyll protoplasts, to gain more insight in the exact location of trehalase. AtTRE1 seemed to be mainly localized in the plasma-membrane (Fig. 10). This result was recently confirmed and published by Frison and coworkers (2007).

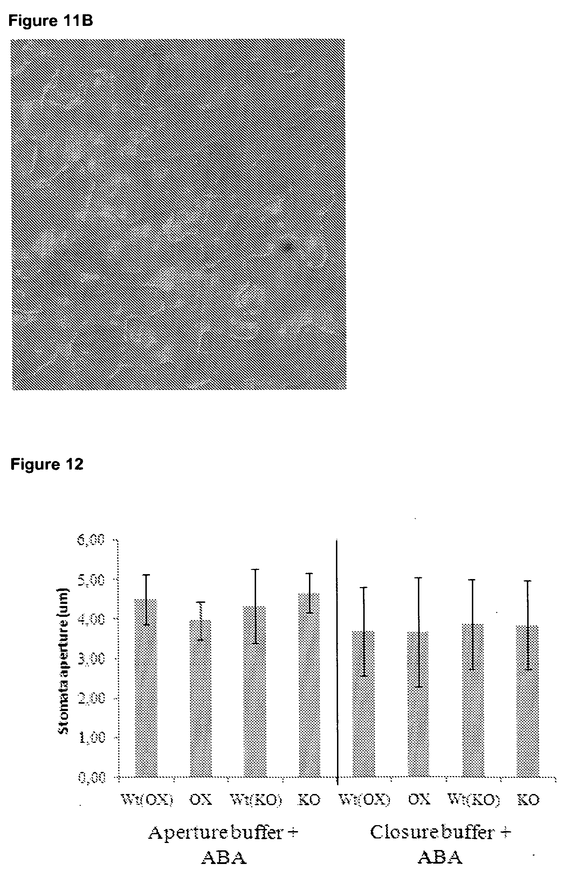

The expression was further analyzed by fusing the trehalase promoter to a GUS/GFP construct and analyzing the plants after GUS staining. In leafs and leaf primordia, a clear specificity of the expression is seen in the stomatal guard cells (Figure 11); expression could also be detected in the flowers (in stomata in sepals of inflorescence, stomata in pedicel of inflorescence , petal veins and chalaza in siliques) and in the root tips.

### Example 5: trehalose expression controls number and opening of stomata

To analyze further the effect of modulation of trehalase expression on stomata, mutants of AtTRE1 were grown in soil under normal moist conditions in a growth room, 21 days after sowing Leaf 1 or Leaf 2 were cut out of the plant and submerged for the abaxial side in aperture buffer (10mM MES, 10mM CaCL2, 10mM KCI, pH 6.1) under light conditions, or closure buffer (10mM MES, 10mM CaCL2, pH 6.1) under dark conditions, after 2 hours leaves were submerged in aperture buffer containing 20uM ABA under light conditions for 2 hours, the abaxial epidermis was mounted on a slide with double-sided tape and stomata aperture measurements (width) were recorded under a microscope, n=50. Trehalase overexpression plants showed a decrease in opening of stomata in aperture buffer, whereas in closure buffer no significant difference between the wild types, overexpression of KO plant could be detected (Figure 12).

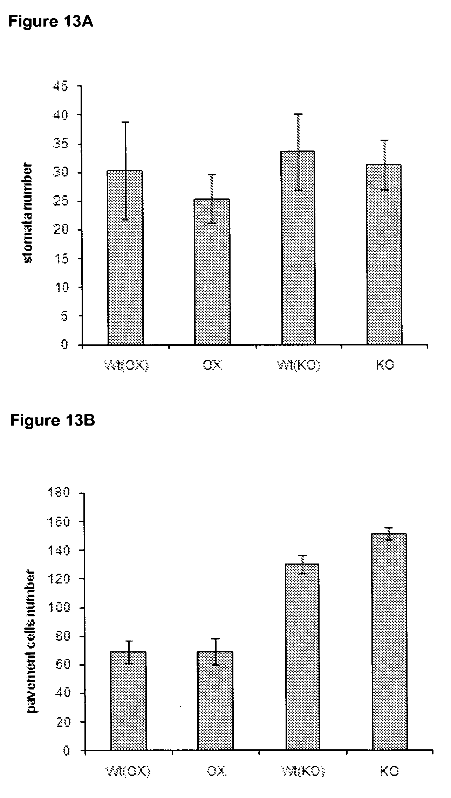

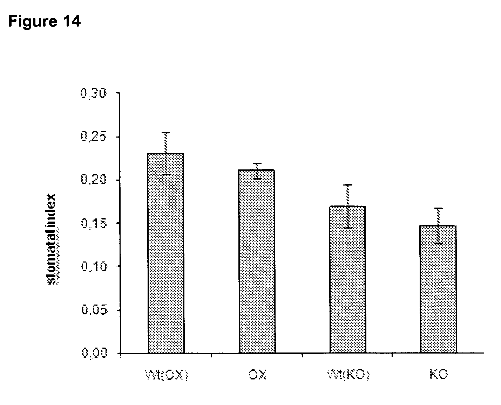

To analyze the effect of trehalase expression on number of stomata, mutants of *AtTRE1* were grown on ½ MS, 1% sucrose horizontal plates, under 16h light/8h dark regime for 21 days. Leaf 1 or Leaf 2 were cut off and cleared in lactic acid for 2 days under constant shaking, leaves were put on a slide and pictures (0.142mm²) of 2 areas (tip and base) of each leaf were taken to count stomata and pavement cells, n=10 and to calculate the stomatal index. The stomatal index corresponds to the ratio of stomata per total number of cells in the epidermis (#stomata/(2x #stomata+#pavement cells). Compared with their respective wild types, there is a slight decrease in number of stomata (Figure 13, A) and in stomatal index (Figure 14). Whereas for the overexpressing plant no influence on the number of pavement cells was seen, the KO showed a small increase in pavement cell number (Figure 13 B). The results indicate that the trehalase modulates drought resistance by modulating the opening of the stomatal guard cells, and/or the number of stomatal guard cells per surface unit and/or the stomatal index.

### REFERENCES

Alonso, J.M., Stepanova, A.N., Leisse, T.J., Kim, C.J., Chen, H., Shinn, P., Stevenson, D.K., Zimmerman, J., Barajas, P., Cheuk, R., Gadrinab, C., Heller, C., Jeske, A., Koesema, E., Meyers, C.C., Parker, H., Prednis, L., Ansari, Y., Choy, N., Deen, H., Geralt, M., Hazari, N., Hom, E., Karnes, M., Mulholland, C., Ndubaku, R., Schmidt, I., Guzman, P., Aguilar-Henonin, L., Schmid, M., Weigel, D., Carter, D.E., Marchand, T., Risseeuw, E., Brogden, D., Zeko, A., Crosby, W.L., Berry, C.C., and Ecker, J.R. (2003). Genome-wide insertional mutagenesis of Arabidopsis thaliana. Science 301, 653-657**.**
Avonce, N., Leyman, B., Mascorro-Gallardo, J.O., Van Dijck, P., Thevelein, J.M., and Iturriaga, G. (2004). The Arabidopsis trehalose-6-P synthase AtTPS1 gene is a regulator of glucose, abscisic acid, and stress signaling. Plant Physiol 136, 3649-3659.
Boyes, D.C., Zayed, A.M., Ascenzi, R., McCaskill, A.J., Hoffman, N.E., Davis, K.R., and Gorlach, J. (2001). Growth stage-based phenotypic analysis of Arabidopsis: a model for high throughput functional genomics in plants. Plant Cell 13, 1499-1510.
Del Sal, G., Manfioletti, G., and Schneider, C. (1988). The CTAB-DNA precipitation method: a common mini-scale preparation of template DNA from phagemids, phages or plasmids suitable for sequencing. Biotechniques 7, 514-520.
Frison, M., Parrou, J.L., Guillaumot, D., Masquelier, D., Francois, J., Chaumont, F., and Batoko, H: (2007). The Arabidopsis thaliana trehalase is a plasma membrane-bound enzyme with extracellular activity. FEBS Lett 581, 4010-4016.
Hauser, M.T., Morikami, A., and Benfey, P.N. (1995). Conditional root expansion mutants of Arabidopsis. Development 121, 1237-1252.
Hwang, I., and Sheen, J. (2001). Two-component circuitry in Arabidopsis cytokinin signal transduction. Nature 413, 383-389.
Ishikawa, R., shirouzu, K., nakashita, H., Teraoka, T., and Arie, T. (2007). Control efficacy of validamycin A against Fusarium wilt correlated with the severity of phytotoxic necrosis formed on tomato tissues. J. Pestic. Sci. 32**.**
Ishikawa, R., Shirouzu, K., Nakashita, H., Lee, H.Y., Motoyama, T., Yamaguchi, I., Teraoka, T., and Arie, T. (2005). Foliar Spray of Validamycin A or Validoxylamine A Controls Tomato Fusarium Wilt. Phytopathology 95, 1209-1216.
Jang, I.C., Oh, S.J., Seo, J.S., Choi, W.B., Song, S.I., Kim, C.H., Kim, Y.S., Seo, H.S., Choi, Y.D., Nahm, B.H. and Kim, J.K. (2003). Expression of bifunctional fusion of Escherichia coli gene for trehalose-6-phosphate synthase and trehalose-6-phosphate phosphatase in transgenic rice plants increases trehalose accumulation and abiotic stress tolerance without stunting growth. Plant Physiology 131, 516-524.
Kovtun, Y., Chiu, W.L., Tena, G., and Sheen, J. (2000). Functional analysis of oxidative stress-activated mitogen-activated protein kinase cascade in plants. Proc Natl Acad Sci U S A 97, 2940-2945.
Kovtun, Y., Chiu, W.L., Zeng, W., and Sheen, J. (1998). Suppression of auxin signal transduction by a MAPK cascade in higher plants. Nature 395, 716-720.
Liu, Y.G., Mitsukawa, N., Oosumi, T., and Whittier, R.F. (1995). Efficient isolation and mapping of Arabidopsis thaliana T-DNA insert junctions by thermal asymmetric interlaced PCR. Plant J 8, 457-463.
Lowry, O.H., Rosebrough, N.J., Farr, A.L., and Randall, R.J. (1951). Protein measurement with the Folin phenol reagent. J Biol Chem 193, 265-275.
Martienssen, R. (1998). Functional genomics: probing plant gene function and expression with transposons. Proc. Natl. Acad. Sci. 95, 2021-2026.
Müller, J., Aeschbacher, R.A., Wingler, A., Boller, T., and Wiemken, A. (2001 a). Trehalose and trehalase in Arabidopsis. Plant Physiol 125, 1086-1093.
Müller, J., Boller, T., and Wiemken, A. (1995b). Effects of validamycin A, a potent trehalase inhibitor, and phytohormones on trehalose metabolism in roots and nodules of soybean and cowpea. Planta 197, 362-368.
Ramon, M., Rolland, F., Thevelein, J.M., Van Dijck, P., and Leyman, B. (2007). ABI4 mediates the effects of exogenous trehalose on Arabidopsis growth and starch breakdown. Plant Mol Biol 63, 195-206.
Schluepmann, H., Pellny, T., van Dijken, A., Smeekens, S., and Paul, M. (2003). Trehalose 6-phosphate is indispensable for carbohydrate utilization and growth in Arabidopsis thaliana. Proc Natl Acad Sci U S A 100, 6849-6854.
Suarez, R., Wong, A., Ramirez, M., Barraza, A., Orozco Mdel, C., Cevallos, M.A., Lara, M., Hernandez, G and Iturriaga, G. (2008). Improvement of drought resistance and grain yield in common bean by overexpressing trehalose-6-phosphate synthase in rhizobia. Mol. Plant Microbe Interact. 21, 958-966.
Sundaresan, V., Springer, P., Volpe, T., Haward, S., Jones, J.D., Dean, C., Ma, H., and Martienssen, R. (1995). Patterns of gene action in plant development revealed by enhancer trap and gene trap transposable elements. Genes Dev 9, 1797-1810.
Wingler, A., Fritzius, T., Wiemken, A., Boller, T., and Aeschbacher, R.A. (2000). Trehalose induces the ADP-glucose pyrophosphorylase gene, ApL3, and starch synthesis in Arabidopsis. Plant Physiol 124, 105-114.
Ye, G.N., Stone, D., Pang, S.Z., Creely, W., Gonzalez, K., and Hinchee, M. (1999). Arabidopsis ovule is the target for Agrobacterium in planta vacuum infiltration transformation. Plant J 19, 249-257.
Yoo, S.D., Cho, Y. ans Sheen, J. (2007). Arabidopsis mesophyll protoplasts: a versatile cell system for transient gene expression analysis. Nature protocols 2, 1565-1572.

## Claims

1. The use of recombinant trehalase overexpression to obtain drought and/or salt tolerance in plants.

2. The use of recombinant trehalase overexpression according to claim 1 to obtain improved growth upon rewatering after a drought period.

3. The use of recombinant trehalase overexpression according to claim 1 or 2, whereby said overexpression is specific for the stomatal guard cells.

4. The use of recombinant trehalase overexpression to modulate the function of the stomatal guard cells.

5. The use of recombinant trehalase overexpression according to claim 4, whereby said modulation of the stomatal function is a limitation of the opening of the stomata.

## Patentansprüche

1. Verwendung von rekombinanter Trehalase-Überexpression, um Trockenheits- und/oder Salztoleranz in Pflanzen zu erzielen.

2. Verwendung von rekombinanter Trehalase-Überexpression nach Anspruch 1, um verbessertes Wachstum bei Wiederbewässerung nach einer Trockenperiode zu erzielen.

3. Verwendung von rekombinanter Trehalase-Überexpression nach Anspruch 1 oder 2, wobei die Überexpression für die Schließzellen spezifisch ist.

4. Verwendung von rekombinanter Trehalase-Überexpression, um die Funktion der Schließzellen zu modulieren.

5. Verwendung von rekombinanter Trehalase-Überexpression nach Anspruch 4, wobei die Modulation der Stomatafunktion eine Einschränkung der Öffnung der Stomata ist.

## Revendications

1. Utilisation de la surexpression de tréhalase recombinée pour obtenir une tolérance à la sécheresse et/ou au sel dans des plantes.

2. Utilisation de la surexpression de tréhalase recombinée selon la revendication 1 pour obtenir une croissance améliorée suite à un réarrosage après une période de sécheresse.

3. Utilisation de la surexpression de tréhalase recombinée selon la revendication 1 ou 2, moyennant quoi ladite surexpression est spécifique aux cellules de garde stomatiques.

4. Utilisation de la surexpression de tréhalase recombinée pour moduler la fonction des cellules de garde stomatiques.

5. Utilisation de la surexpression de tréhalase recombinée selon la revendication 4, moyennant quoi ladite modulation de la fonction stomatique est une limitation de l'ouverture des stomates.


### SEQUENCE LISTING

<110> VIB VZW KATHOLIEKE UNIVERSITEIT LEUVEN, K.U.LEUVEN R&D
<120> USE OF TREHALASE TO OBTAIN DROUGHT RESISTANCE IN PLANTS
<130> PVD/TRE/V308
<150> US 61/207,188
   <151> 2009-02-09
<160> 18
<170> PatentIn version 3.5
<210> 1
   <211> 34
   <212> DNA
   <213> Artificial Sequence
<220>
   <223> Primer
<400> 1
   ggaagatcta tgaaatcata caaacttaat aacc 34
<210> 2
   <211> 31
   <212> DNA
   <213> Artificial Sequence
<220>
   <223> Primer
<400> 2
   tcccccgggg gcttcaatgc taagatgaga g 31
<210> 3
   <211> 31
   <212> DNA
   <213> Artificial Sequence
<220>
   <223> Primer
<400> 3
   aactatcttg tctacaagaa aagtgggact a 31
<210> 4
   <211> 31
   <212> DNA
   <213> Artificial Sequence
<220>
   <223> Primer
<400> 4
   tagtcccact tttcttgtag acaagatagt t 31
<210> 5
   <211> 19
   <212> DNA
   <213> Artificial Sequence
<220>
   <223> Primer
<400> 5
   acatggaagg ctgagaacc 19
<210> 6
   <211> 20
   <212> DNA
   <213> Artificial Sequence
<220>
   <223> Primer
<400> 6
   ccaagaaata atccactacg 20
<210> 7
   <211> 21
   <212> DNA
   <213> Artificial Sequence
<220>
   <223> Primer
<400> 7
   cctctcatct tagcattgaa g 21
<210> 8
   <211> 22
   <212> DNA
   <213> Artificial Sequence
<220>
   <223> Primer
<400> 8
   ccaagaaata atccactacg at 22
<210> 9
   <211> 20
   <212> DNA
   <213> Artificial Sequence
<220>
   <223> Primer
<400> 9
   agcgagagag aaagcgtttc 20
<210> 10
   <211> 20
   <212> DNA
   <213> Artificial Sequence
<220>
   <223> Primer
<400> 10
   ccttccatgt ctcagattcc 20
<210> 11
   <211> 20
   <212> DNA
   <213> Artificial Sequence
<220>
   <223> Primer
<400> 11
   gaagagcacc ctgttcttct 20
<210> 12
   <211> 20
   <212> DNA
   <213> Artificial Sequence
<220>
   <223> Primer
<400> 12
   tggcgtacaa ggagagaaca 20
<210> 13
   <211> 22
   <212> DNA
   <213> Artificial Sequence
<220>
   <223> Primer
<400> 13
   tggttcacgt agtgggccat cg 22
<210> 14
   <211> 21
   <212> DNA
   <213> Artificial Sequence
<220>
   <223> Primer
<400> 14
   ccgtcccgca agttaaatat g 21
<210> 15
   <211> 24
   <212> DNA
   <213> Artificial Sequence
<220>
   <223> Primer
<400> 15
   gcctttttca aactcaagtt gcac 24
<210> 16
   <211> 24
   <212> DNA
   <213> Artificial Sequence
<220>
   <223> Primer
<400> 16
   gagtttatta tgtgtgtgcg tgga 24
<210> 17
   <211> 20
   <212> DNA
   <213> Artificial Sequence
<220>
   <223> Primer
<400> 17
   actcatctcc acgcacacac 20
<210> 18
   <211> 20
   <212> DNA
   <213> Artificial Sequence
<220>
   <223> Primer
<400> 18
   aatctcgaac cgggaatgat 20